(19) Europäisches Patentamt
European Patent Office
Office européen des brevets

(11) EP 4 785 779 A1

(12) **EUROPEAN PATENT APPLICATION**
published in accordance with Art. 153(4) EPC

(43) Date of publication:
**05.08.2026 Bulletin 2026/32**

(21) Application number: **24872536.8**

(22) Date of filing: **27.09.2024**

(51) International Patent Classification (IPC):
***A01H 1/00*** *(2006.01)* ***A01H 5/00*** *(2018.01)*
***A01H 6/00*** *(2018.01)* ***A01H 6/08*** *(2018.01)*
***A01H 6/82*** *(2018.01)* ***C12N 5/10*** *(2006.01)*
***C12N 15/54*** *(2006.01)*

(52) Cooperative Patent Classification (CPC):
**A01H 1/00; A01H 1/06; A01H 5/00; A01H 6/00; A01H 6/08; A01H 6/82; C12N 5/04; C12N 5/10;** C12N 9/10

(86) International application number:
**PCT/JP2024/034732**

(87) International publication number:
**WO 2025/070755 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.09.2023 JP 2023170210**

(71) Applicant: **Suntory Flowers Limited**
**Tokyo 108-0014 (JP)**

(72) Inventors:
- **MISATO, Tomoya**
**Higashiomi-shi, Shiga 527-0138 (JP)**
- **YAMADA, Masahiro**
**Higashiomi-shi, Shiga 527-0138 (JP)**
- **MATSUI, Keisuke**
**Higashiomi-shi, Shiga 527-0138 (JP)**
- **TSUJI, Takuya**
**Soraku-gun, Kyoto 619-0284 (JP)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

# (54) PLANT OF WHICH FLOWER COLOR IS CHANGED, AND METHOD FOR PRODUCING SAME

(57) There is provided a plant of which flower color is changed. A method for producing a plant, the method including: accumulating an anthocyanidin pigment in a cell of a petal of the plant in an amount of 30 mass% or more relative to a total amount of pigments present in the cell, wherein a flower color of the plant after the accumulation of the anthocyanidin pigment is changed so that a value of b* in a CIE $L^*a^*b^*$ color system is decreased, as compared with a flower color of the plant before the accumulation of the anthocyanidin pigment.

Fig. 12

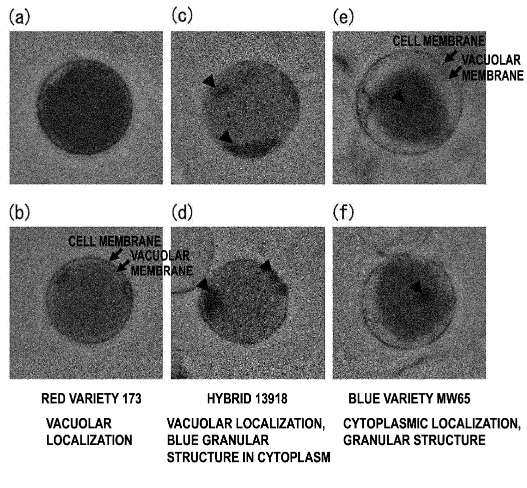


EP 4 785 779 A1

## Description

### Technical Field

The present invention relates to a plant with a changed flower color and a method for producing the same.

### Background Art

The flower industry is striving to develop novel and various varieties. One effective method for breeding a novel variety is to change flower color, and classical breeding methods have been used to produce varieties that exhibit various flower colors for most commercial varieties.

For example, Patent Literature 1 reports a method for producing a plant having a blue-based flower color. More specifically, the method of Patent Literature 1 is a method for producing a plant having a blue-based flower color, characterized by including allowing a delphinidin-type anthocyanin in which 3' and 5' positions of an anthocyanin B ring are glycosylated and a flavone glycoside or a flavonol glycoside to coexist in cells of the plant.

In addition, Patent Literature 2 discloses a novel plant of the genus Mandevilla having a novel flower color tone that has not been able to be produced so far. More particularly, the novel plant of the genus Mandevilla of Patent Literature 2 is a plant of the genus Mandevilla containing at least one kind of carotenoid pigment in a petal, characterized in that the carotenoid pigment is neoxanthin or a derivative thereof.

### Citation List

#### Patent Literature

PTL 1: WO 2017/169699
PTL 2: JP 2021-175402 A

#### Non Patent Literature

NPL 1: Tatsuzawa, F. et. al. Biochem. Systemat. Ecol. 99, 2021, 104347
NPL 2: Macz-Pop, G. A. et. al. Food Chem. 94, 2006, 448-456
NPL 3: Oyama, K. et. al. J. Agric. Food Chem. 63, 2015, 7630-7635

### Summary of Invention

#### Technical Problem

There is still a need for development of a plant with a changed flower color.

For example, the plant of the genus Mandevilla includes only varieties having white, pink, and red flower colors, and Patent Literature 2 provides a plant of the genus Mandevilla with a novel color containing a carotenoid pigment, but studies on blue-colored plants of the genus Mandevilla are still required.

The present invention is intended to improve the above circumstances, and an object thereof is to provide a plant with a changed flower color and a method for producing the same.

#### Solution to Problem

The present invention that achieves the above object is as follows.

Aspect 1

A method for producing a plant, the method including:

accumulating an anthocyanidin pigment in a cell of a petal of the plant in an amount of 30 mass% or more relative to a total amount of pigments present in the cell, wherein

a flower color of the plant after the accumulation of the anthocyanidin pigment is changed so that a value of $b^*$ in a CIE $L^*a^*b^*$ color system is decreased, as compared with a flower color of the plant before the accumulation of the anthocyanidin pigment.

Aspect 2

The method according to Aspect 1, wherein the anthocyanidin pigment is accumulated by disrupting at least a portion of an anthocyanidin modification enzyme gene.

Aspect 3

The method according to Aspect 2, further including:

disrupting at least a portion of a promoter region of the anthocyanidin modification enzyme gene, wherein
the anthocyanidin modification enzyme is selected from an anthocyanidin glycosylation enzyme gene and/or an anthocyanidin 3-glucoside glycosylation enzyme.

Aspect 4

The method according to Aspect 3, wherein

the anthocyanidin glycosylation enzyme is a galactosyltransferase, and
at least one sequence selected from the group consisting of a sequence shown in SEQ ID NO: 1, a sequence shown in SEQ ID NO: 2, and a sequence having at least 90% identity thereto is deleted in a promoter region of a gene of the galactosyltransferase.

Aspect 5

The method according to Aspect 3, wherein

the anthocyanidin 3-glucoside glycosylation enzyme is a xylosyltransferase, and
at least one sequence selected from the group consisting of a sequence shown in SEQ ID NO: 3 and a sequence having at least 90% identity thereto is inserted into a promoter region of a gene of the xylosyltransferase.

Aspect 6

The method according to any one of Aspects 1 to 5, wherein the anthocyanidin pigment is cyanidin.

Aspect 7

The method according to any one of Aspects 1 to 6, wherein the anthocyanidin pigment is localized in a cytoplasm of the cell.

Aspect 8

The method according to any one of Aspects 1 to 7, wherein chlorogenic acid and aluminum ions are contained in the cell of the petal of the plant.

Aspect 9

The method according to any one of Aspects 1 to 8, wherein the plant is at least one selected from the group consisting of a plant of the family Apocynaceae, a plant of the family Solanaceae, a plant of the family Linderniaceae, and a plant of the family Scrophulariaceae.

Aspect 10

The method according to any one of Aspects 1 to 9, wherein the plant is at least one selected from the group consisting of a plant of the genus Mandevilla, a plant of the genus Petunia, and a plant of the genus Torenia.

Aspect 11

The method according to Aspect 10, wherein the plant is a plant with an accession number FERM BP-22483, which has been deposited with the National Institute of Technology and Evaluation, International Patent Organism Depositary (NITE-IPOD) under a receipt number FERM ABP-22483, or a progeny of the plant.

Aspect 12

A plant wherein, in a cell of a petal of the plant,

an anthocyanidin pigment is accumulated in an amount of 30 mass% or more relative to a total amount of pigments present in the cell, and
at least a portion of an anthocyanidin modification enzyme gene is deleted.

Aspect 13

The plant according to Aspect 12, wherein the anthocyanidin pigment is localized in a cytoplasm of the cell.

Aspect 14

The plant according to Aspect 12 or 13, wherein

at least a part of a promoter region of the anthocyanidin modification enzyme gene is deleted, and
the anthocyanidin modification enzyme is selected from an anthocyanidin glycosylation enzyme and/or an anthocyanidin 3-glucoside glycosylation enzyme.

Aspect 15

The plant according to Aspect 14, wherein

the anthocyanidin glycosylation enzyme is a galactosyltransferase, and
at least one sequence selected from the group consisting of a sequence shown in SEQ ID NO: 1, a sequence shown in SEQ ID NO: 2, and a sequence having at least 90% identity thereto is deleted in a promoter region of a gene of the galactosyltransferase.

Aspect 16

The plant according to Aspect 14, wherein

the anthocyanidin 3-glucoside glycosylation enzyme is a xylosyltransferase, and
at least one sequence selected from the group consisting of a sequence shown in SEQ ID NO: 3 and a sequence having at least 90% identity thereto is inserted into a promoter region of a gene of the xylosyltransferase.

Aspect 17

The plant according to any one of Aspects 12 to 16, wherein the anthocyanidin pigment is cyanidin.

Aspect 18

The plant according to any one of Aspects 12 to 17, wherein chlorogenic acid and aluminum ions are contained in the cell of the petal of the plant.

Aspect 19

The plant according to any one of Aspects 12 to 18, wherein the plant is at least one selected from the group consisting of a plant of the family Apocynaceae, a plant of the family Solanaceae, a plant of the family Linderniaceae, and a plant of the family Scrophulariaceae. Aspect 20

The plant according to any one of Aspects 12 to 19, wherein the plant is at least one selected from the group

consisting of a plant of the genus Mandevilla, a plant of the genus Petunia, and a plant of the genus Torenia.

Aspect 21

The plant according to Aspect 20, wherein the plant of the genus Mandevilla is a plant with an accession number FERM BP-22483, which has been deposited with the National Institute of Technology and Evaluation, International Patent Organism Depositary (NITE-IPOD) under a receipt number FERM ABP-22483, or a progeny of the plant.

Aspect 22

A progeny of the plant described in any one of Aspects 12 to 21.

Aspect 23

A propagation material of the plant described in any one of Aspects 12 to 21.

Aspect 24

A plant part, tissue, or cell of the plant described in any one of Aspects 12 to 21.

Advantageous Effects of Invention

According to the present invention, it is possible to provide a plant of which flower color is changed.

Brief Description of Drawings

[Fig. 1] Fig. 1 is a diagram illustrating results of pigment analysis in Example 1.

[Fig. 2] Fig. 2 is a diagram illustrating results of HPLC analysis in Example 1.

[Fig. 3] Fig. 3 is a diagram illustrating results of structural analysis in Example 2.

[Fig. 4] Fig. 4 is a diagram illustrating expression analysis of a galactosyltransferase gene and a xylosyltransferase gene in Example 3.

[Fig. 5] Fig. 5 is a diagram illustrating results of LC-MS analysis in Example 4.

[Fig. 6] Fig. 6 is a diagram illustrating results of extraction with various solvents in Example 5.

[Fig. 7] Fig. 7 is a diagram illustrating an experiment of Example 6.

[Fig. 8] Fig. 8 is a diagram illustrating analysis results and alignment of a base sequence of a galactosyltransferase gene in Example 7.

[Fig. 9] Fig. 9 is a diagram illustrating analysis results and alignment of a base sequence of a xylosyltransferase gene in Example 7.

[Fig. 10] Fig. 10 is a diagram illustrating a design for preparing a gene marker in Example 7.

[Fig. 11] Fig. 11 is a diagram illustrating results of PCR amplification in Example 7.

[Fig. 12] Fig. 12 is a diagram illustrating intracellular localization of a pigment in Example 8.

Description of Embodiments

Hereinafter, embodiments of the present invention will be described in detail. The present invention is not limited

to the following embodiments, and can be variously modified within the scope of the present invention.

Method for Producing Plant

A method for producing a plant of the present invention (hereinafter, also simply referred to as "the method of the present invention") is a method for producing a plant, including:

accumulating an anthocyanidin pigment in a cell of a petal of the plant in an amount of 30 mass% or more relative to a total amount of pigments present in the cell,
wherein a flower color of the plant after the accumulation of the anthocyanidin pigment is changed so that a value of $b^*$ in a CIE $L^*a^*b^*$ color system is decreased, as compared with a flower color of the plant before the accumulation of the anthocyanidin pigment.

As described in Patent Literature 1, there has been a technical idea of accumulating an anthocyanin pigment or the like in a glycoside form in a petal cell. In addition, since it is known that a pigment in an aglycone state is generally unstable and is stabilized in a cell by being subjected to modification such as glycosylation or acylation, there has been no report on a technical idea of accumulating an anthocyanidin pigment, that is, a pigment in an aglycone state in a petal cell, or on a precedent example in which an anthocyanidin pigment was accumulated in a cell of a petal of a plant.

Therefore, the technical idea of accumulating an anthocyanidin pigment in an amount of 30 mass% or more relative to a total amount of pigments present in a cell of a petal of a plant, according to the present invention, is novel, and results of Examples 1 to 8 described later suggest that the method of the present invention enables accumulation of an anthocyanidin pigment in a cell (particularly, in a cytoplasm) of a petal of a plant.

According to the method of the present invention, an anthocyanidin pigment can be accumulated in a cell of a petal of a plant in an amount of 30 mass% or more relative to a total amount of pigments present in the cell. More specifically, an amount of the anthocyanidin pigment accumulated can be 30 mass% or more, 35 mass% or more, 40 mass% or more, 45 mass% or more, 50 mass% or more, 55 mass% or more, 60 mass% or more, 65 mass% or more, 70 mass% or more, 75 mass% or more, 80 mass% or more, 85 mass% or more, 90 mass% or more, 95 mass% or more, 99 mass% or more, or about 100 mass%, relative to the total amount of the pigments present in the cell. The amount of the anthocyanidin pigment accumulated and the total amount of the pigments present in the cell can be determined by, for example, extracting the pigments from a lyophilized petal with a mixed solution of trifluoroacetic acid and acetonitrile and performing HPLC analysis. More specifically, reference may be made to Examples 1 and 2 described later. An upper limit value of the amount of the anthocyanidin pigment accumulated is not particularly limited, and may be, for example, 100 mass%, 99.9 mass%, or 99 mass% relative to the total amount of the pigments present in the cell.

In the present invention, an amount of the anthocyanidin pigment in the plant after the accumulation of the anthocyanidin pigment is not particularly limited, and may be, for example, 0.05 mg or more, 0.1 mg or more, 0.2 mg or more, 0.3 mg or more, 0.4 mg or more, 0.5 mg or more, 0.6 mg or more, 0.7 mg or more, 0.8 mg or more, 0.9 mg or more, 1.0 mg or more, 1.0 mg or more, 1.2 mg or more, 1.3 mg or more, 1.4 mg or more, 1.5 mg or more, or 2.0 mg or more, and may be 2.5 mg or less, 2.0 mg or less, 1.8 mg or less, 1.5 mg or less, or 1.1 mg or less, per g of petal.

In the present invention, a change in flower color of the plant before and after the accumulation of the anthocyanidin pigment can be determined by a $b^*$ value in a CIE $L^*a^*b^*$ color system as an indicator. More specifically, a flower color of the plant after the accumulation of the anthocyanidin pigment may be changed so that the value of $b^*$ in the CIE $L^*a^*b^*$ color system is decreased as compared with a flower color of the plant before the accumulation of the anthocyanidin pigment.

Here, the CIE $L^*a^*b^*$ color system is a color system standardized by the International Commission on Illumination (CIE), is widely known as a notation representing color tones, and is also referred to as CIE1976 ($L^*a^*b^*$) color system. In the $L^*a^*b^*$ color system, lightness is represented by $L^*$, and chromaticity indicating hue and chroma is represented by $a^*$ and $b^*$. The $a^*$ and $b^*$ respectively indicate directions of colors. The $a^*$ indicates a red direction, $-a^*$ indicates a green direction, the $b^*$ indicates a yellow direction and $-b^*$ indicates a blue direction. The values of $L^*$, $a^*$, and $b^*$ can be obtained from tristimulus values X, Y, Z from the following equations:

$$L^* = 116(Y/Y_0)^{1/3} - 16;$$

$$a^* = 500[(X/X_0)^{1/3} - (Y/Y_0)^{1/3}];$$

and

$$b^* = 200[(Y/Y_0)^{1/3} - (Z/Z_0)^{1/3}]$$

where $X/X_0$, $Y/Y_0$ and $Z/Z_0$ are > 0.008856, where $X_0$, $Y_0$ and $Z_0$ represent tristimulus values of a standard light source.

The analysis of color according to the CIE $L^*a^*b^*$ color system can be easily performed by an integrating sphere type spectrophotometer, and for example, a commercially available spectrophotometer (CM-2022, Konica Minolta, Inc.) can be used.

According to the method of the present invention, the decrease in the value of $b^*$ means that the flower color of the plant after the accumulation of the anthocyanidin pigment is shifted to a more blue color system and changed. Here, a degree of decrease in the value of $b^*$ is not particularly limited, and may be, for example, 5.0 or more, 10.0 or more, 15.0 or more, 20.0 or more, 25.0 or more, 30.0 or more, 35.0 or more, 40.0 or more, 45.0 or more, or 50.0 or more.

In the present invention, the value of $b^*$ of the flower color of the plant after the accumulation of the anthocyanidin pigment is not particularly limited, and may be, for example, 20.0 or less, 15.0 or less, 10.0 or less, 5.0 or less, 1.0 or less, or 0 or less. The value of $b^*$ may be preferably less than 0. When the value of $b^*$ is less than 0, more specifically, an absolute value thereof may be, for example, 1.0 or more, 5.0 or more, 10.0 or more, 15.0 or more, 20.0 or more, 25.0 or more, or 30.0 or more.

In one embodiment, according to the method of the present invention, when a plant of the genus Mandevilla having a red flower color is used, the flower color of the plant of the genus Mandevilla after the accumulation of the anthocyanidin pigment can be changed so that the value of $b^*$ in the CIE $L^*a^*b^*$ color system is decreased from "20.9" to "-30.9", that is, shifted to the blue side, as compared with the flower color (red) of the plant of the genus Mandevilla before the accumulation of the anthocyanidin pigment.

The plant which can be applied to the method of the present invention is not particularly limited, and may be, for example, at least one selected from the group consisting of a plant of the family Apocynaceae, a plant of the family Solanaceae, a plant of the family Linderniaceae, and a plant of the family Scrophulariaceae.

Furthermore, the plant applicable to the method of the present invention may be at least one selected from the group consisting of a plant of the genus Mandevilla, a plant of the genus Petunia, and a plant of the genus Torenia, but is not limited thereto.

In general, it is known that anthocyanin pigments are contained in plants and that red, blue, and purple flower colors are exhibited due to the anthocyanin pigments. An anthocyanin pigment is obtained by adding a sugar to an anthocyanidin (aglycone) pigment by a glycosylation enzyme or the like.

In the present invention, the anthocyanidin pigment is an aglycone of an anthocyanin pigment, and has a structure of a compound represented by the following General Formula (I):

[Chem. 1]

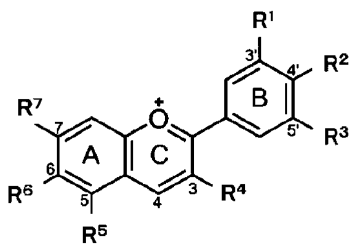

(I)

(in Formula (I), $R^1$, $R^2$, $R^3$, $R^4$, $R^5$, $R^6$, and $R^7$ may each independently be a hydrogen atom, an - OH group, or an $-OCH_3$ group).

In General Formula (I), the anthocyanidin is classified into three groups, i.e., pelargonidin, cyanidin and delphinidin, according to the number of hydroxy groups of the B ring of the anthocyanidin moiety. Various anthocyanin pigments can be present depending on the type and number of sugars added to the A ring and the C ring of the anthocyanidin moiety.

In the method of the present invention, the anthocyanidin pigment may be cyanidin. The cyanidin has a structure represented by the following Formula (II).

[Chem. 2]

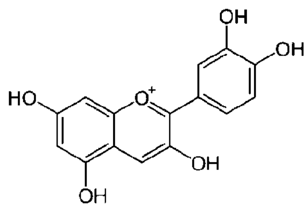

( I I )

In the method of the present invention, for example, when a plant of the genus Mandevilla having a red flower color is used, it is preferable to accumulate cyanidin in the cell of the petal of the plant. This can change the flower color of the plant of the genus Mandevilla after the accumulation of cyanidin to blue, as compared with the flower color (red) of the plant of the genus Mandevilla before the accumulation of cyanidin.

In the present invention, the anthocyanidin pigment may be in a form of being localized in the cytoplasm of the cell of the petal of the plant, and can be in a state of being localized in the cytoplasm of epidermal cells of the petal of the plant, in particular. In contrast, the anthocyanin pigment is generally localized in vacuoles in the cell of the petal. The localization of the pigment (i.e., location where the pigment exists) in the cell can be confirmed by, for example, preparing a protoplast from a petal, and observing the protoplast with a phase contrast microscope using a glass slide for counting blood cells.

In the method of the present invention, the anthocyanidin pigment can be accumulated by disrupting at least a portion of an anthocyanidin modification enzyme gene in the cell of the petal of the plant. The anthocyanidin modification enzyme may be an enzyme capable of modifying an anthocyanidin in an aglycone state, and may be, for example, an enzyme for glycosylation of an anthocyanidin or an enzyme for acylation of an anthocyanidin.

In one aspect of the present invention, the anthocyanidin modification enzyme may be selected from anthocyanidin glycosylation enzymes and/or anthocyanidin 3-glucoside glycosylation enzymes. In this case, in the method of the present invention, the anthocyanidin pigment can be accumulated by disrupting at least a portion of the anthocyanidin glycosylation enzyme gene and/or the anthocyanidin 3-glucoside glycosylation enzyme gene, and preferably, the anthocyanidin pigment can be accumulated by disrupting at least a portion of both the anthocyanidin glycosylation enzyme gene and the anthocyanidin 3-glucoside glycosylation enzyme gene.

Without being limited to theory, for example, by disrupting at least a portion of the anthocyanidin glycosylation enzyme gene and the anthocyanidin 3-glucoside glycosylation enzyme gene in the cell of the petal of the plant, the anthocyanidin pigment cannot be converted to anthocyanin, which is a glycoside thereof, and as a result, the anthocyanidin pigment can be accumulated. A site of disruption of the anthocyanidin glycosylation enzyme gene and the anthocyanidin 3-glucoside glycosylation enzyme gene is not particularly limited, and may be, for example, their promoter regions.

The disruption of the anthocyanidin modification enzyme gene may be carried out by a routine technique in the art. For example, mutagenesis by irradiation with gamma rays, heavy ion beams, or the like, RNAi, genome editing, or the like may be used. More specifically, the disruption can be achieved by applying, for example, deletion, insertion, substitution, and/or addition to at least a portion of the DNA sequence of the anthocyanidin modification enzyme gene.

In the method of the present invention, the anthocyanidin glycosylation enzyme may be specifically, for example, a galactosyltransferase. A gene of the galactosyltransferase may have a DNA sequence shown in SEQ ID NO: 4 and an amino acid sequence shown in SEQ ID NO: 5. The anthocyanidin 3-glucoside glycosylation enzyme may be, specifically, for example, a xylosyltransferase. A gene of the xylosyltransferase may have a DNA sequence shown in SEQ ID NO: 6 and an amino acid sequence shown in SEQ ID NO: 7.

For example, when a galactosyltransferase and a xylosyltransferase are present for cyanidin, the cyanidin can be converted to a cyanidin glycoside represented by the following Formula (III) through a two-stage saccharification reaction. At the first stage, the cyanidin is converted to cyanidin 3-galactoside by addition of galactose to the 3-position thereof by the galactosyltransferase. At the second stage, the cyanidin 3-galactoside can be further added with xylose by the xylosyltransferase.

[Chem. 3]

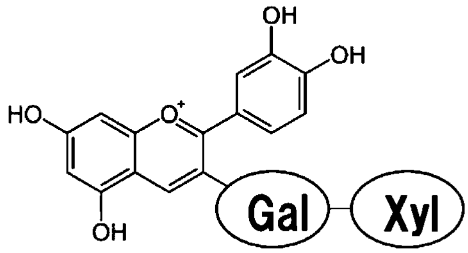

**cyanidin 3-*O*-[2-*O*-(xylosyl)-galactoside]**

(III)

(In Formula (III), Gal represents galactose, and Xyl represents xylose.)

In the method of the present invention, when the anthocyanidin glycosylation enzyme is a galactosyltransferase, at least one sequence selected from the group consisting of a sequence shown in SEQ ID NO: 1, a sequence shown in SEQ ID NO: 2, and a sequence having at least 90% identity thereto may be deleted in a promoter region of a gene of the galactosyltransferase.

In addition, in order to identify whether or not the sequence is deleted in the galactosyltransferase gene, for example, PCR primers may be designed so as to span the deleted portion as compared with the case of a gene whose sequence is not deleted. Then, PCR is performed using the designed primer, and when an amplified fragment having a target length is obtained, it can be identified that the sequence is deleted.

In the method of the present invention, when the anthocyanidin 3-glucoside glycosylation enzyme is a xylosyltransferase, at least one sequence selected from the group consisting of a sequence shown in SEQ ID NO: 3 and a sequence having at least 90% identity thereto may be inserted into a promoter region of a gene of the xylosyltransferase.

In addition, for identification as to whether or not the sequence is inserted, in the xylosyltransferase, for example, a PCR primer may be designed from the inserted portion, as compared with the case of a gene in which the sequence is not inserted. Then, PCR is performed using the designed primer, and when an amplified fragment having a target length is obtained, it can be identified that the sequence is inserted.

In the present invention, the term "identity" means the amount (number) of amino acid residues or bases constituting two chains of polypeptide sequences (or amino acid sequences) or polynucleotide sequences (or base sequences) that can be determined to be identical in the matching relationship between the two chains, and means a degree of sequence correlation between two polypeptide sequences or two polynucleotide sequences. The "identity" can be easily calculated. There are many known methods for measuring identity between two polynucleotide sequences or polypeptide sequences, and the term "identity" is well known to those skilled in the art (see, e.g., Lesk, A. M. (Ed.), Computational Molecular Biology, Oxford University Press, New York, (1988); Smith, D. W. (Ed.), Biocomputing: Informatics and Genome Projects, Academic Press, New York, (1993); Grifin, A. M. & Grifin, H. G. (Ed.), Computer Analysis of Sequence Data: Part I, Human Press, New Jersey, (1994); von Heinje, G., Sequence Analysis in Molecular Biology, Academic Press, New York, (1987); and Gribskov, M. & Devereux, J. (Ed.), Sequence Analysis Primer, M-Stockton Press, New York, (1991)).

In addition, unless otherwise specified, the numerical value of "identity" described in the present description may be a numerical value calculated using an identity retrieval program known to those skilled in the art, and is preferably a numerical value calculated using the ClustalW program of the MacVector application (version 9.5 Oxford Molecular Ltd., Oxford, England). In the present invention, the degree of "identity" between sequences may be, for example, about 90% or more, 91% or more, 92% or more, 93% or more, or 94% or more, preferably 95% or more, more preferably 96% or more, further preferably 97% or more, particularly preferably 98% or more, and most preferably 99% or more.

In the present invention, chlorogenic acid and aluminum ions may be contained in the cell of the petal of the plant. The presence of chlorogenic acid and aluminum ions can lead to a stronger change in flower color due to a copigment effect. For example, when a plant of the genus Mandevilla having a red flower color is used, the presence of chlorogenic acid and aluminum ions in the cell of the petal of the plant allows the flower color of the plant of the genus Mandevilla after the accumulation of cyanidin to be more strongly changed to blue, as compared with the flower color (red) of the plant of the genus Mandevilla before the accumulation of cyanidin.

Plant

The present invention also provides a plant. The plant of the present invention is a plant, wherein, in a cell of a

petal of the plant,

an anthocyanidin pigment is accumulated in an amount of 30 mass% or more relative to a total amount of pigments present in the cell, and

at least a portion of an anthocyanidin modification enzyme gene is deleted.

The plant of the present invention may be produced by the method of the present invention described above. Therefore, for the description regarding the anthocyanidin modification enzyme, reference may be made to the description regarding the method of the present invention as appropriate.

In addition, at least a portion of the promoter region of the anthocyanidin modification enzyme gene may be deleted in the plant of the present invention. The anthocyanidin modification enzyme may be selected from anthocyanidin glycosylation enzymes and/or anthocyanidin 3-glucoside glycosylation enzymes. When the anthocyanidin glycosylation enzyme is a galactosyltransferase, at least one sequence selected from the group consisting of a sequence shown in SEQ ID NO: 1, a sequence shown in SEQ ID NO: 2, and a sequence having at least 90% identity thereto may be deleted in a promoter region of a gene of the galactosyltransferase. When the anthocyanidin 3-glucoside glycosylation enzyme is a xylosyltransferase, at least one sequence selected from the group consisting of a sequence shown in SEQ ID NO: 3 and a sequence having at least 90% identity thereto may be inserted into a promoter region of a gene of the xylosyltransferase.

The anthocyanidin pigment of the plant of the present invention can be localized in the cytoplasm of the cell of the petal of the plant. In the plant of the present invention, the anthocyanidin pigment may be cyanidin.

The plant of the present invention may be at least one selected from the group consisting of a plant of the family Apocynaceae, a plant of the family Solanaceae, a plant of the family Linderniaceae, and a plant of the family Scrophulariaceae, and, more specifically, may be at least one selected from the group consisting of a plant of the genus Mandevilla, a plant of the genus Petunia, and a plant of the genus Torenia.

The plant of the genus Mandevilla according to the present invention can be produced from a plant of the genus Mandevilla with an accession number FERM BP-22483, which has been deposited with the National Institute of Technology and Evaluation, International Patent Organism Depositary (NITE-IPOD) under a receipt number FERM ABP-22483, or a progeny thereof, using a routine technique in plant breed improvement. Such a routine technique may include, for example, known propagation techniques such as vegetative propagation and seed propagation, as well as tissue culture techniques for culturing plant cells, tissues or organs, and genetic recombination techniques capable of directly introducing a useful trait. The plant of the genus Mandevilla according to the present invention can be produced by vegetative propagation of a plant of the genus Mandevilla (accession number: FERM BP-22483; based on Budapest Treaty), which has been deposited with the National Institute of Technology and Evaluation, International Patent Organism Depositary (NITE-IPOD) under a receipt number FERM ABP-22483 on September 22, 2023, or a progeny thereof. As the "vegetative propagation", for example, a method such as cutting (including leaf cutting), layering, stem layering (marcottage method), division, or grafting, as well as a clone seedling prepared by tissue culture can be used, but cutting is particularly preferable.

The "cutting" is an asexual propagation method in which a portion of a plant that has neither a stem nor a root is cut out and an independent individual plant having a stem and a root is prepared therefrom, and examples thereof include modes such as branch cutting, trunk cutting (stem cutting), root cutting, and leaf cutting depending on the portion to be used. The cutting is typically performed by inserting a part of a branch or stem of a parent plant into soil, and leaving a portion of the part exposed above the ground. In the present invention, a site used for the "cutting" is not particularly limited.

In addition, for cutting, a bud sport of the plant of the genus Mandevilla with an accession number FERM BP-22483, which has been deposited with the National Institute of Technology and Evaluation, International Patent Organism Depositary (NITE-IPOD) under a receipt number FERM ABP-22483, or a progeny thereof, may be used. The term "bud sport" means a phenomenon in which a flower, a new bud, a leaf, or the like is different from a genetic trait possessed by the individual due to a mutation of a growth point or the like with respect to only a certain branch of a plant, or an individual who has expressed such a phenomenon. In plants, the body is formed sequentially from the growth point, and therefore the mutated portion is distinguished from the non-mutated portion, and its characteristics may be fixed as traits. Thus, by cutting using a bud sport branch having at least one changed flower, novel varieties of the plant of the genus Mandevilla having other characteristics can be produced while maintaining desired characteristics (e.g., blue flower color).

In another aspect of the present invention, the present invention provides a propagation material (vegetative propagation material such as a cutting, a seed, or the like), a plant part (flower, stem, branch, leaf, root, or the like), a tissue, or a cell of the above-described plant of the genus Mandevilla or a progeny thereof.

In yet another aspect of the present invention, the present invention provides a product derived from the above-described plant of the genus Mandevilla or a progeny thereof, typically a potted plant, a cut flower, or a processed product produced from the cut flower.

In the present description, the term "progeny" includes self-propagated or outbred progeny of the plant of the genus Mandevilla with an accession number FERM BP-22483, which has been deposited with the National Institute of

Technology and Evaluation, International Patent Organism Depositary (NITE-IPOD) under a receipt number FERM ABP-22483, and includes not only progeny of the first generation, but also progeny of all generations, of the plant of the genus Mandevilla with the accession number FERM BP-22483, which has been deposited with the National Institute of Technology and Evaluation, International Patent Organism Depositary (NITE-IPOD) under the receipt number FERM ABP-22483. Furthermore, in the present description, the term "progeny" includes those produced from a parent Mandevilla plant based on a routine technique in plant breed improvement, and may include, for example, those produced by known propagation techniques such as vegetative propagation and seed propagation of a parent plant, as well as tissue culture techniques for culturing plant cells, tissues, or organs, genetic recombination techniques capable of directly introducing useful traits and the like, and mutants such as a bud sport of a parent Mandevilla plant. Such progeny preferably has the desired characteristics derived from the parent plant of the genus Mandevilla.

Examples

Hereinafter, the present invention will be described more specifically with reference to Examples, but is not limited to these Examples.

Example 1

In Example 1, the pigment composition contained in the petal cells of mandevilla of the blue variety (MW65) having a blue flower color, mandevilla of the red variety (173) having a red flower color, and a hybrid (13918) of these mandevillas, which were held by Suntory Flowers Co., Ltd., was analyzed by HPLC. Here, the hybrid (13918) is obtained by crossing the blue variety (MW65) as a mother and a variety of a different line from the red variety (173) as a father.

Note that, color analysis according to the CIE $L^*a^*b^*$ color system of each of the above varieties (blue variety, red variety, and hybrid thereof) was performed using a commercially available spectrophotometer (CM-2022, Konica Minolta, Inc.). The results obtained are shown in Table 1 below.

[Table 1]

| Analysis of color according to CIE $L^*a^*b^*$ color system | $L^*$ | $a^*$ | $b^*$ |
|---|---|---|---|
| Red variety (173) | 22.7 | 43.4 | 20.9 |
| Hybrid (13918) | 32.9 | 23.9 | -15.8 |
| Blue variety (MW65) | 37.9 | 19.6 | -30.9 |

NPL 1 reports that cyanidin is the main anthocyanidin in petal cells of plants of the genus Mandevilla. Therefore, the anthocyanin pigments in the petals of each of the above varieties (blue variety, red variety, and hybrid thereof) were analyzed as follows.

Preparation of Test Liquid

To 0.5 mg of each lyophilized petal was added 4 mL of 0.1%TFA/50% acetonitrile, and the anthocyanin pigment was extracted ultrasonically for 20 min.

The glycosides were analyzed by HPLC of the extract as it was.

In the case of analysis of aglycones excluding sugars and the like, the above extract was evaporated to dryness overnight in a desiccator, dissolved in 6 N HCl, saponified in a boiling water bath for 20 minutes, extracted with amyl alcohol, and then analyzed by HPLC.

HPLC Analysis

As an analysis instrument, Shimadzu Prominence HPLC system (LC-20AD, SIL-20AC, CBM-20A, CTO-20AC, SPD-M20A, SPD-20A) was used.

For the analysis of the glycosides, RSpak DE-413L (250 mm × 4.6 mm ID, Shodex) was used as a column; for feeding LC mobile phases, 0.5% TFA/water and 0.5% TFA-containing 50% CH3CN/water were used as mobile phases A and B, respectively; the mobile phase B concentration was changed from 20% to 100% over 15 minutes by a binary gradient; and then analysis was performed at the mobile phase B concentration of 100% and a flow rate of 0.4 mL/min for 10 minutes.

In the case of the analysis of the aglycones, YMC-Pack ODS-A (150 mm × 6 mm ID, YMC) was used as a column; acetic acid/methanol/water (15/17.5/67.5, vol/vol) was used for feeding an LC mobile phase; and analysis was performed

at a flow rate of 1.0 mL/min.

Identification and quantification of peaks were performed by using cyanidin, delphinidin, pelargonidin, malvidin, peonidin, and petunidin as standard substances.

As a result, as illustrated in Fig. 1, it was found that cyanidin was the main anthocyanidin in both the blue variety (MW65) and the red variety (173) in the samples from which sugars were removed by acid hydrolysis. This result is consistent with the report of NPL 1.

As illustrated in Fig. 2, in the analysis of the samples with sugars, specifically high peaks (peak A and peak B) were detected in the red variety (173) and the blue variety (MW65), respectively. Both the peaks (peak A and peak B) were detected in the hybrid (13918). In Example 2 shown below, these peaks were subjected to structural analysis.

Example 2

The structures of the peak A and the peak B in the red variety (173) and the blue variety (MW65) of Example 1 were estimated by LC-MS.

More specifically, petal extracts were prepared using 5% AcOH, 50% MeCN-5% AcOH, and MeCN-5% AcOH. In the LC-MS experiment, LCMS-IT-TOF (Shimadzu) was used as an instrument; YMC-Triart C18 (3 $\mu$m, 2.1 $\times$ 100 mm, YMC) was used as a column; 0.1% formic acid/water and 0.1% formic acid/acetonitrile were used as mobile phases A and B, respectively; the mobile phase B concentration was changed from 20% to 100% over 15 minutes by a binary gradient; and then analysis was performed at the mobile phase B concentration of 100% and a flow rate of 0.4 mL/min for 10 minutes. The peak of anthocyanin was extracted from the chromatogram at 530 nm, and structural analysis was performed based on the spectra of MS and

MS/MS.

As a result, as illustrated in Fig. 3, it was found that the peak A of the red variety (173) was cyanidin 3-O-[2-O-(xylosyl)-galactoside] as described in NPL 1.

On the other hand, it was surprisingly found that the peak B of the blue variety (MW65) was cyanidin without any modification (aglycone) = anthocyanidin (Fig. 3). There have been almost no known previous cases where aglycones were highly accumulated in plant bodies, and this is an analysis result that overturns existing common knowledge.

In addition, NPL 2 reports that anthocyanidins accumulate in the pericarp of some varieties of kidney beans, but the proportion thereof is 22%. In contrast, it was found that cyanidin shown by the peak B was accumulated in an amount of 70 mass% or more relative to the total amount of the pigments in the cytoplasms and vacuoles of the petal cells of the blue variety (MW65).

Example 3

In the blue variety (MW65), it was considered that anthocyanidins were highly accumulated due to the decrease in the expression or enzymatic activity of galactosyltransferase genes and xylosyltransferase genes in petals, which are considered to be necessary for the synthesis of cyanidin 3-O-[2-O-(xylosyl)-galactoside] from cyanidin aglycone.

Therefore, the galactosyltransferase gene and xylosyltransferase gene in mandevilla were first identified as follows. Glycosyltransferase genes were listed from the group of genes estimated from the mandevilla genome sequence, and the amino acid sequences of the proteins encoded by them were aligned with the sequence of the previously reported anthocyanidin glycosyltransferase by ClustalW, and a phylogenetic tree was created by ETE3. From the obtained phylogenetic tree, genes considered to be orthologs of the galactosyltransferase genes and xylosyltransferase genes of other plants that had been reported previously were estimated. The expression of the group of genes was analyzed by the RNA-seq method and the RT-PCR method.

RNA-seq Method

Total RNA was extracted from mandevilla petals with RNeasy Plant mini (Qiagen) after pretreatment with Fruit-mate for RNA Purification (Takara Bio Inc.). A library for RNA-Seq was adjusted using MGIEasy RNA Directional Library Prep Set (MGI). Paired end sequences of 150 bases were obtained using MGI DNBSeq-G400RS. After removal of low quality sequences and removal of adaptor sequences, the expression level was calculated by mapping to the reference sequence.

Quantitative RT-PCR Method

The total RNA was subjected to a reverse transcription reaction using ReverTra Ace (Toyobo Co., Ltd.) to prepare

cDNA. Quantitative PCR was performed using PowerUp SYBR Green Master Mix (Applied Biosystems) and StepOnePlus real-time PCR system (Applied Biosystems). The expression level was calculated using an actin gene as an endogenous control.

As a result, as illustrated in Fig. 4, both the genes (galactosyltransferase genes and xylosyltransferase genes) were highly expressed in the red variety (173), but the expression of these genes was hardly observed in the blue variety (MW65). The results also showed that the blue-based mandevilla was consistent with the idea that most of cyanidins were not modified with sugars and were present in the form of aglycones.

Example 4

It is known that aglycones are generally unstable and are stabilized in cells by being subjected to modification such as glycosylation or acylation. One of possibilities of stabilizing aglycones of highly accumulated cyanidins is a copigment effect (see, for example, NPL 3). The copigment effect is well known in hydrangea, and is an effect of increasing a blue tint by combining anthocyanins with substances such as flavones (these substances are almost colorless or pale yellow) and metal ions in vivo. Substances that may act as one of the causes of blue flowers were searched for in the blue variety (MW65) by LC-MS.

(LC-MS)

Petal extracts were prepared using 5% AcOH, 50% MeCN-5% AcOH, and MeCN-5% AcOH. In the LC-MS experiment, LCMS-IT-TOF (Shimadzu) was used as an instrument; YMC-Triart C18 (3 $\mu$m, 2.1 $\times$ 100 mm, YMC) was used as a column; 0.1% formic acid/water and 0.1% formic acid/acetonitrile were used as mobile phases A and B, respectively; the mobile phase B concentration was changed from 20% to 100% over 15 minutes by a binary gradient; and then analysis was performed at the mobile phase B concentration of 100% and a flow rate of 0.4 mL/min for 10 minutes. A large peak was found in the blue-based mandevilla near 6.3 minutes in the chromatogram at 325 nm. The structure of this peak was analyzed based on the spectra of

MS and MS/MS.

As a result, as illustrated in Fig. 5, it was found that chlorogenic acid (3-CQA) was accumulated at a high concentration in the blue variety (MW65). It was suggested that this chlorogenic acid (3-CQA) may contribute to blue color development of the blue variety (MW65).

Example 5

When 2 g of the petal of the blue variety mandevilla was squeezed with a garlic squeezer, a squeezed liquid exhibiting a red color was obtained at pH 3.7 (see Fig. 6 (a)). This result suggested possibilities that cyanidin aglycone exhibits a red color at a low pH, and that cyanidin aglycone needs to be localized in the cytoplasm in order to develop a blue color.

When the petal of the blue variety mandevilla was treated with various solvents, all the pigments were extracted into methanol in the case of amphiphilic methanol extraction, the petal turned white, and all the pigments were extracted (see Fig. 6 (b)). This is considered to be due to the extraction of both cyanidin aglycone and glycosides, and the methanol containing the pigment turned blue.

On the other hand, in the extraction using hexane (hydrophobic), which is an organic solvent, the petal turned red although hexane was colorless and transparent (see Fig. 6 (c)). This is considered to be because the aglycone of the fat-soluble component was extracted, but the glycoside remained, and the petal turned red.

Example 6

In vitro pigment reconstitution experiments were performed to look at color development under neutral conditions mimicking the cytoplasmic environment. Cyanidin chloride (ChromaDex) and 3-O-Caffeoylquinic Acid (Nagara Science Co., Ltd.) were dissolved in DMSO so as to achieve 10 mM. The reconstitution experiment using cyanidin alone was performed by adding the above-described cyanidin chloride solution to a 50 mM phosphate buffer solution having a pH of from 4.4 to 9.3 so as to achieve 0.5 mM, mixing them together, and reacting them at room temperature. The reconstitution experiment under the presence of copigment and metal ion was performed by adding the above-described cyanidin chloride solution and 3-O-Caffeoylquinic acid solution to a 50 mM phosphate buffer solution having a pH of from 4.4 to 9.3 so as to achieve 0.5 mM, adding an aluminum ion solution (1 mM ammonium aluminum sulfate solution) so as to achieve an aluminum ion concentration of 0.1 mM, mixing them together, and reacting them at room temperature (see the table in Fig.

7).

As a result, it was observed that cyanidin aglycone alone developed a blue color at a neutral pH (see Fig. 7). This strongly suggests that cyanidin aglycone alone, when present in the cytoplasm, can develop a blue color without the action of copigment or metal ions.

Example 7

In order to prepare a blue variety (MW65)-specific marker, the above-described galactosyltransferase genes and xylosyltransferase genes and the base sequences in the vicinity thereof were analyzed. As a result, it was found that the deletion of two sites, 16 bp and 30 bp, in the promoter region of the galactosyltransferase genes and the insertion of 173 bp in the promoter region of the xylosyltransferase genes were specifically present in the blue variety (see Figs. 8 and 9). Two sequences derived from the paternal and maternal alleles were obtained from the red variety (173), and thus named 173-h1 and 173-h2, respectively. Thus, the galactosyltransferase genes of the red variety 173-h1 have the sequence shown in SEQ ID NO: 8, and the galactosyltransferase genes of the red variety 173-h2 have the sequence shown in SEQ ID NO: 9. The xylosyltransferase genes of the red variety 173-h1 have the sequence shown in SEQ ID NO: 10, and the xylosyltransferase genes of the red variety 173-h2 have the sequence shown in SEQ ID NO: 11.

For the galactosyltransferase genes, PCR primers were designed so as to span the deleted portion (g21571-F7 (SEQ ID NO: 12): ACTGGCCTCGCCATTAACG, g21571-R5 (SEQ ID NO: 13): GTAATTTAATTAAGATGCGTAATTCT CTG) (see Fig. 10).

In addition, for the xylosyltransferase genes, PCR primers were designed within the insertion site (g19028-F7 (SEQ ID NO: 14): AGTGTCTCTTCCTTAGTTCCTG, g19028-R5 (SEQ ID NO: 15): CTGAATTTTAAGAGATCGTTCAT AATACG) (see Fig. 10).

PCR was carried out using the above primers and DNAs extracted from leaves of the blue variety (MW65), the red variety (173), and the hybrid (13918) using NucleoSpin Plant II (MACHEREY-NAGEL) as templates. PCR was performed using Tks gflex DNA polymerase (Takara Bio Inc.) under the conditions of 30 cycles at 98°C for 15 seconds, at 60°C for 20 seconds, and at 68°C for 30 seconds.

As a result, for the galactosyltransferase genes, an amplified fragment of 122 bp was obtained in the blue variety (MW65) and the hybrid (13918), whereas no amplification was observed in the red variety (173) (see Fig. 11 (a)). For the xylosyltransferase genes, an amplified fragment of 121 bp was obtained in the blue variety (MW65) and the hybrid (13918), whereas no amplification was observed in the red variety (173) (see Fig. 11 (b)).

Thus, it was found that the blue variety (MW65) and its progeny could be easily distinguished by performing PCR using these primers and examining whether or not an amplified fragment of the desired length was obtained.

Example 8

To confirm the intracellular localization of the pigment, protoplasts were prepared from petals of the blue variety (MW65), the red variety (173), and the hybrid (13918), and the location of the pigment was confirmed.

The protoplasts were prepared by the following method. To a 100-mL Erlenmeyer flask were added 0.1 g of a petal section and 10 mL of an enzymatic solution (1% (w/v) Cellulase "ONOZUKA" RS (Yakult Pharmaceutical Co., Ltd.), 1% (w/v) Mecerozyme (Yakult Pharmaceutical Co., Ltd.), 180 mM KCl, 20 mM CaCl2, 20 mM MgCl2, pH 5.5), and the mixture was kept warm in an incubator at 30°C for 90 minutes while reciprocally shaking the flask at an amplitude of 40 mm and 50 strokes per minute. The protoplast was transferred to a 50-mL glass round-bottom centrifuge tube, and precipitated by centrifugation at 100 × g (730 rpm) for 2 minutes, and the precipitate was gently suspended in the remaining liquid with a pipette. To the suspension, 10 mL of 0.5M Mannitol was added and suspended, and the suspension was tested as an observation sample. The sample was placed on a slide glass for counting blood cells and observed with a phase contrast microscope BX40 (Olympus).

In the red variety (173), the red pigment was uniformly distributed in the vacuoles (see Figs. 12 (a) and (b)), whereas in the blue variety (MW65), the cytoplasm, not the vacuoles, was observed to be stained blue, and blue granular structures were also observed in some places (see Figs. 12 (e) and (f)).

Both states were observed in the hybrid (13918), and the vacuoles were uniformly stained red, whereas blue granular structures were localized in the cytoplasm (see Figs. 12 (c) and (d)). This revealed that cyanidin aglycone was localized in the cytoplasm, not in the vacuoles where glycosides were localized, and partly formed a granular structure. This cytoplasmic pigment localization is a phenomenon that has not been known so far, and was considered to be a trait that characterizes the blue variety (MW65) and its progeny.

| | | |
|---|---|---|
| 0-1 | Form PCT/RO/134 | |

(continued)

| | | |
|---|---|---|
| 0-1-1 | The indications relating to deposited microorganism or other biological material (PCT Rule 13bis) are prepared by: | JPO-PAS i520 |
| 0-2 | International application number | |
| 0-3 | Applicant or agent document number | P240493WO |

| | | |
|---|---|---|
| 1 | The following indications relate to the microorganism or biological material described in the description. | |
| 1-1 | Paragraph number | 0053 |
| 1-3 | Indication of deposit | |
| 1-3-1 | Name of depository | IPOD, National Institute of Technology and Evaluation, International Patent Organism Depositary (NITE-IPOD) |
| 1-3-2 | Address of depository | #120, 2-5-8, Kazusakamatari, Kisarazu-shi, Chiba, 292-0818 Japan |
| 1-3-3 | Date of deposit | September 22, 2023 (22.09.2023) |
| 1-3-4 | Accession number | IPOD FERM BP-22483 |
| 1-5 | Designated countries for which these indications are made | All designated countries |

Receiving Office use only

| | | |
|---|---|---|
| 0-4 | This form was received with the international application (Yes/No) | ✓ |
| 0-4-1 | Authorized personnel | Yuka Hirakawa |

International Bureau use only

| | | |
|---|---|---|
| 0-5 | The date on which this form was received by the International Bureau | |
| 0-5-1 | Authorized personnel | |

## Claims

**1.** A method for producing a plant, the method comprising:

accumulating an anthocyanidin pigment in a cell of a petal of the plant in an amount of 30 mass% or more relative to a total amount of pigments present in the cell,
wherein a flower color of the plant after the accumulation of the anthocyanidin pigment is changed so that a value of b* in a CIE $L^*a^*b^*$ color system is decreased, as compared with a flower color of the plant before the accumulation of the anthocyanidin pigment.

**2.** The method according to claim 1, wherein the anthocyanidin pigment is accumulated by disrupting at least a portion of an anthocyanidin modification enzyme gene.

**3.** The method according to claim 2, further comprising:

disrupting at least a portion of a promoter region of the anthocyanidin modification enzyme gene, wherein the anthocyanidin modification enzyme is selected from an anthocyanidin glycosylation enzyme and/or an anthocyanidin 3-glucoside glycosylation enzyme.

**4.** The method according to claim 3,

wherein the anthocyanidin glycosylation enzyme is a galactosyltransferase, and
wherein at least one sequence selected from the group consisting of a sequence shown in SEQ ID NO: 1, a sequence shown in SEQ ID NO: 2, and a sequence having at least 90% identity thereto is deleted in a promoter region of a gene of the galactosyltransferase.

**5.** The method according to claim 3,

wherein the anthocyanidin 3-glucoside glycosylation enzyme is a xylosyltransferase, and
wherein at least one sequence selected from the group consisting of a sequence shown in SEQ ID NO: 3 and a sequence having at least 90% identity thereto is inserted into a promoter region of a gene of the xylosyltransferase.

**6.** The method according to any one of claims 1 to 5, wherein the anthocyanidin pigment is cyanidin.

**7.** The method according to any one of claims 1 to 6, wherein the anthocyanidin pigment is localized in a cytoplasm of the cell.

**8.** The method according to any one of claims 1 to 7, wherein chlorogenic acid and aluminum ions are contained in the cell of the petal of the plant.

**9.** The method according to any one of claims 1 to 8, wherein the plant is at least one selected from the group consisting of a plant of the family Apocynaceae, a plant of the family Solanaceae, a plant of the family Linderniaceae, and a plant of the family Scrophulariaceae.

**10.** The method according to any one of claims 1 to 9, wherein the plant is at least one selected from the group consisting of a plant of the genus Mandevilla, a plant of the genus Petunia, and a plant of the genus Torenia.

**11.** The method according to claim 10, wherein the plant of the genus Mandevilla is a plant with an accession number FERM BP-22483, which has been deposited with the National Institute of Technology and Evaluation, International Patent Organism Depositary (NITE-IPOD) under a receipt number FERM ABP-22483, or a progeny of the plant.

**12.** A plant wherein, in a cell of a petal of the plant,

an anthocyanidin pigment is accumulated in an amount of 30 mass% or more relative to a total amount of pigments present in the cell, and
at least a portion of an anthocyanidin modification enzyme gene is deleted.

**13.** The plant according to claim 12, wherein the anthocyanidin pigment is localized in a cytoplasm of the cell.

**14.** The plant according to claim 12 or 13,

wherein at least a portion of a promoter region of the anthocyanidin modification enzyme gene is deleted, and
wherein the anthocyanidin modification enzyme is selected from an anthocyanidin glycosylation enzyme and/or an anthocyanidin 3-glucoside glycosylation enzyme.

**15.** The plant according to claim 14,

wherein the anthocyanidin glycosylation enzyme is a galactosyltransferase, and
wherein at least one sequence selected from the group consisting of a sequence shown in SEQ ID NO: 1, a sequence shown in SEQ ID NO: 2, and a sequence having at least 90% identity thereto is deleted in a promoter region of a gene of the galactosyltransferase.

**16.** The plant according to claim 14,

wherein the anthocyanidin 3-glucoside glycosylation enzyme is a xylosyltransferase, and
wherein at least one sequence selected from the group consisting of a sequence shown in SEQ ID NO: 3 and a sequence having at least 90% identity thereto is inserted into a promoter region of a gene of the xylosyltransferase.

**17.** The plant according to any one of claims 12 to 16, wherein the anthocyanidin pigment is cyanidin.

**18.** The plant according to any one of claims 12 to 17, wherein chlorogenic acid and aluminum ions are contained in the cell of the petal of the plant.

**19.** The plant according to any one of claims 12 to 18, wherein the plant is at least one selected from the group consisting of a plant of the family Apocynaceae, a plant of the family Solanaceae, a plant of the family Linderniaceae, and a plant of the family Scrophulariaceae.

**20.** The plant according to any one of claims 12 to 19, wherein the plant is at least one selected from the group consisting of a plant of the genus Mandevilla, a plant of the genus Petunia, and a plant of the genus Torenia.

**21.** The plant according to claim 20, wherein the plant of the genus Mandevilla is a plant with an accession number FERM BP-22483, which has been deposited with the National Institute of Technology and Evaluation, International Patent Organism Depositary (NITE-IPOD) under a receipt number FERM ABP-22483, or a progeny of the plant.

**22.** A progeny of the plant described in any one of claims 12 to 21.

**23.** A propagation material of the plant described in any one of claims 12 to 21.

**24.** A plant part, tissue, or cell of the plant described in any one of claims 12 to 21.

Fig. 1

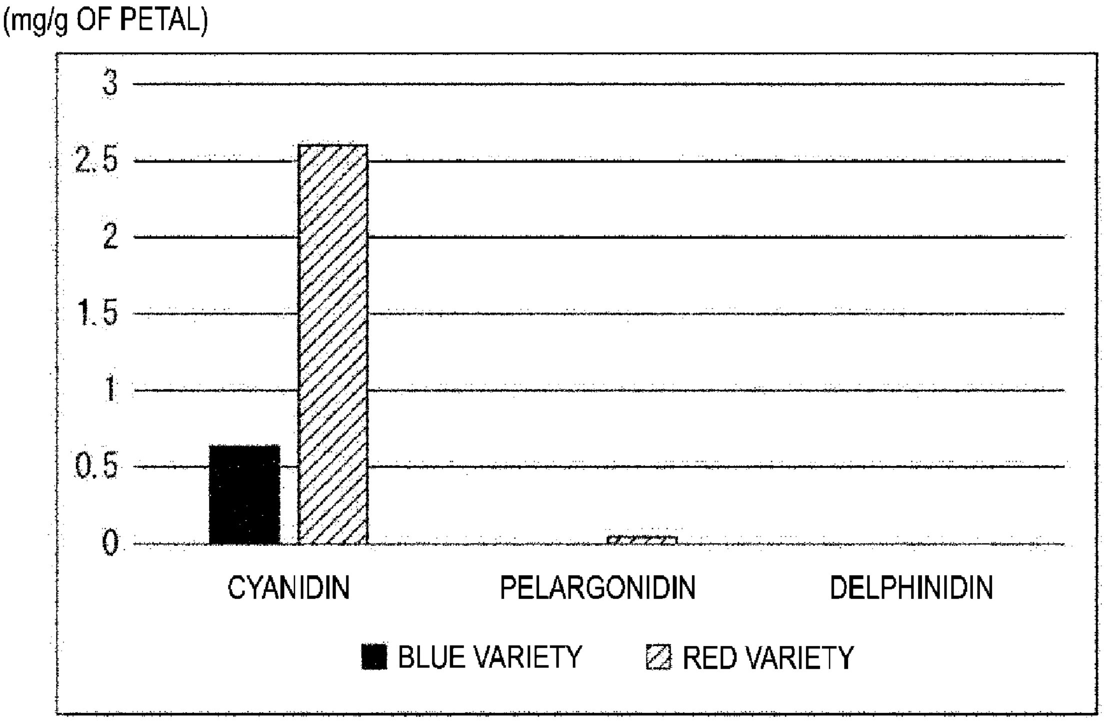


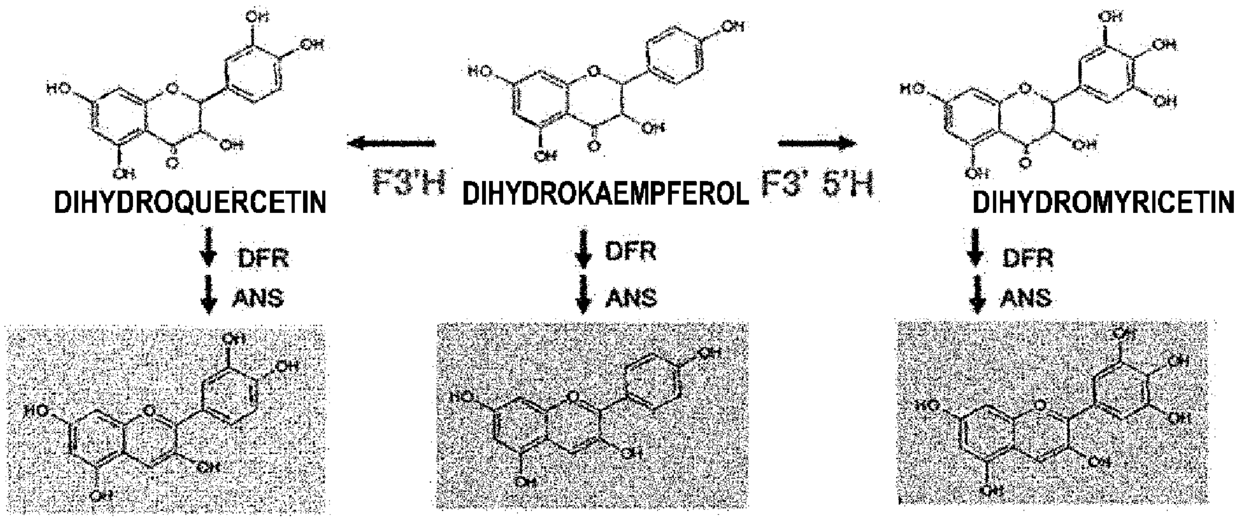


DIHYDROQUERCETIN

DIHYDROKAEMPFEROL

DIHYDROMYRICETIN

CYANIDIN (RED)

PELARGONIDIN (ORANGE)

DELPHINIDIN (BLUE)

Fig. 2

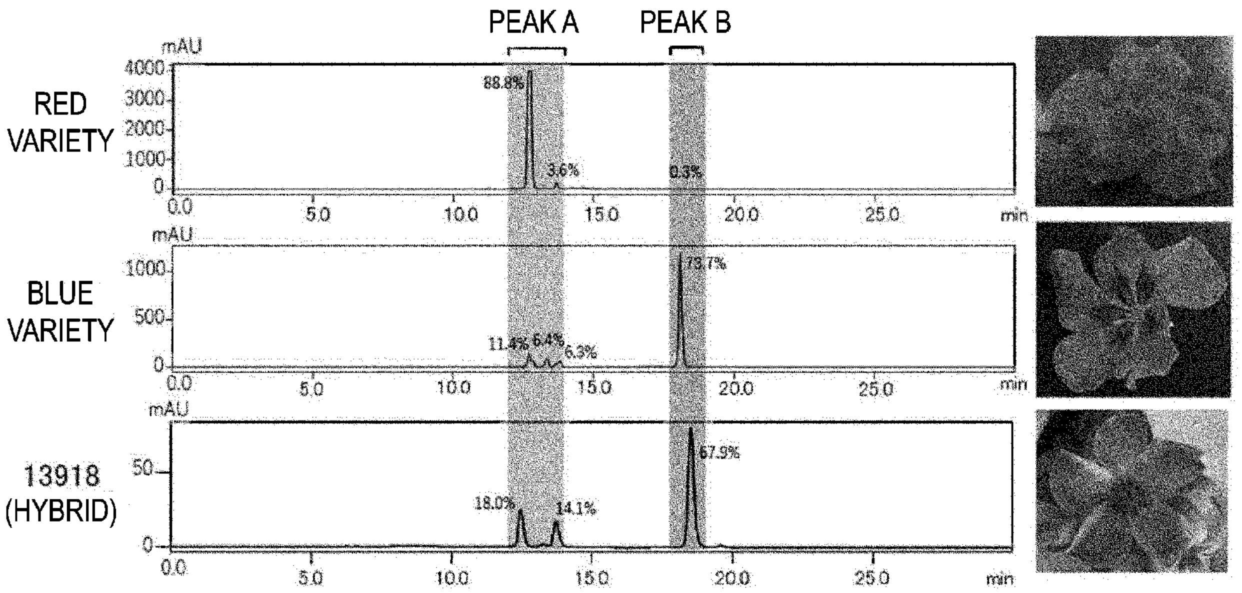
PEAK A
PEAK B
RED VARIETY
BLUE VARIETY
13918 (HYBRID)
mAU
88.8%
3.6%
0.3%
11.4%
6.4%
6.3%
73.7%
18.0%
14.1%
67.9%
min

Fig. 3

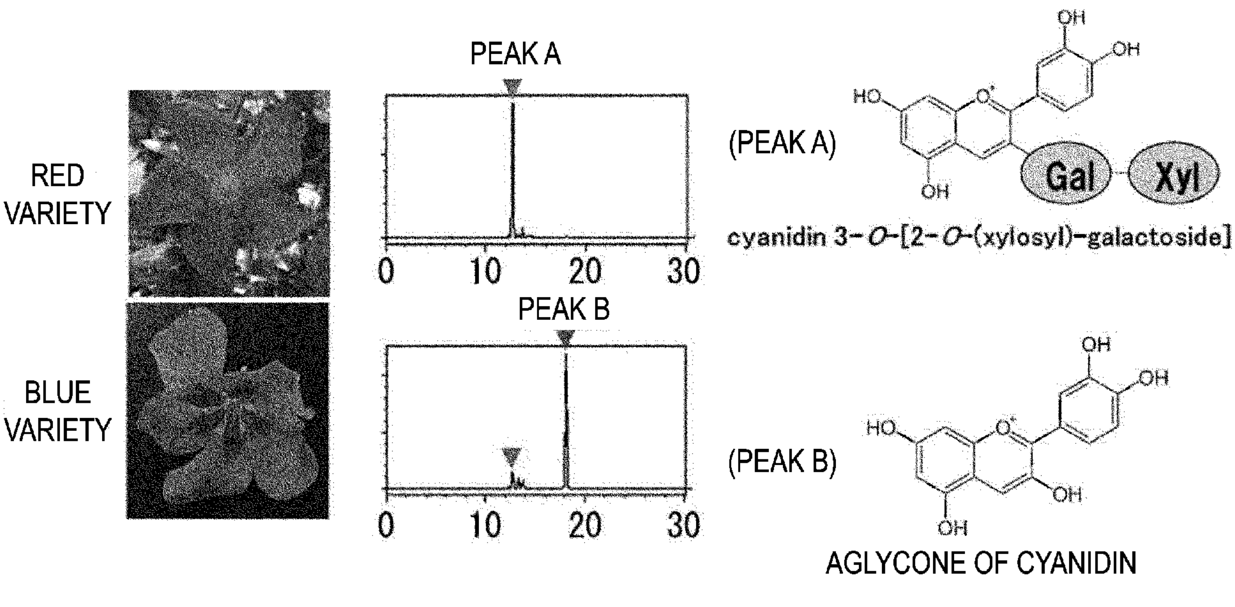
PEAK A
RED VARIETY
0 10 20 30
PEAK B
BLUE VARIETY
0 10 20 30
(PEAK A)
cyanidin 3-O-[2-O-(xylosyl)-galactoside]
(PEAK B)
AGLYCONE OF CYANIDIN

Fig. 4

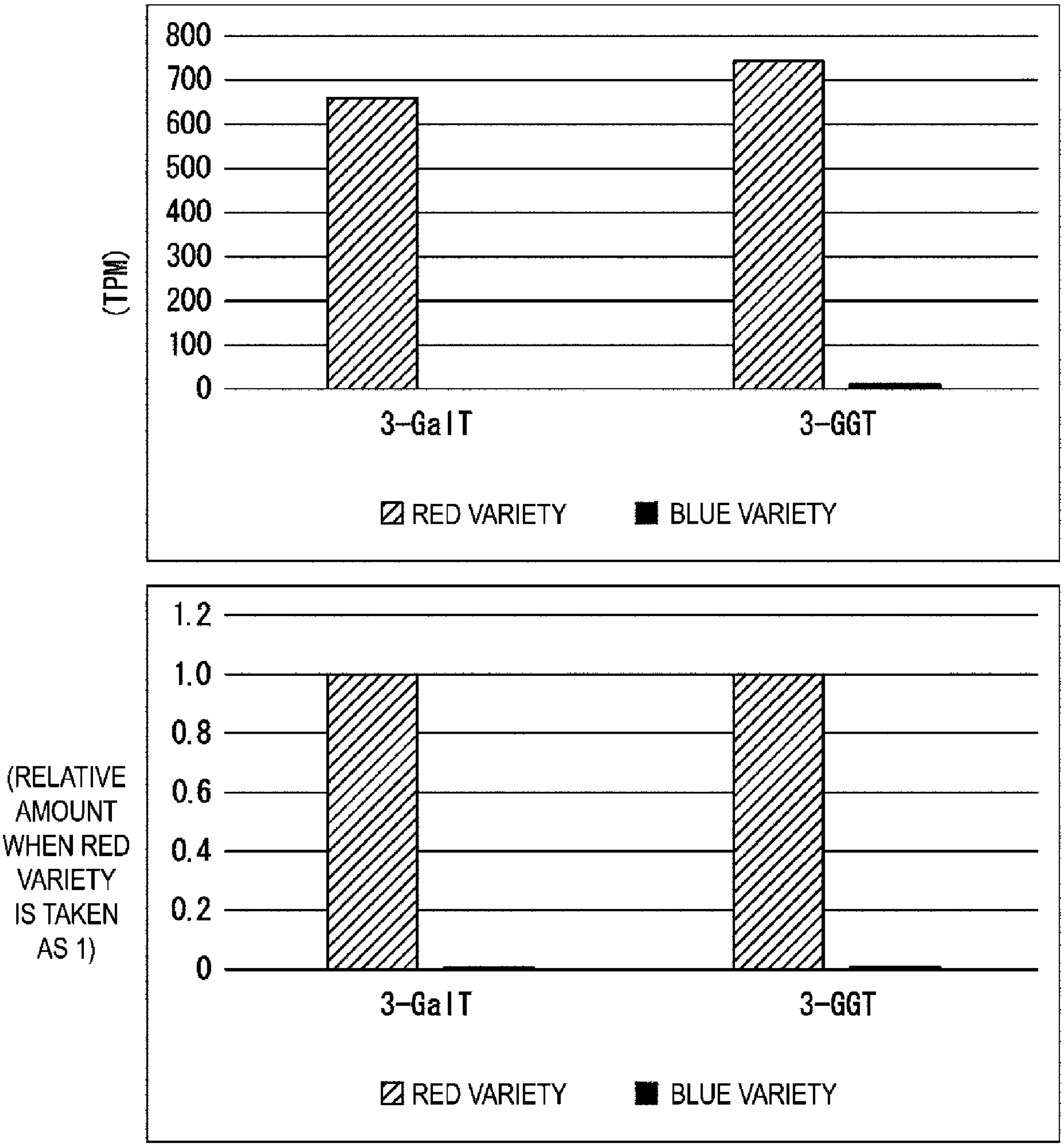


ANALYSIS OF EXPRESSION OF GALACTOSYLTRANSFERASE
3-GalT AND XYLOSYLTRANSFERASE 3-GGT (UPPER: RNA-seq, LOWER: RT-PCR)

# Fig. 5

BLUE VARIETY

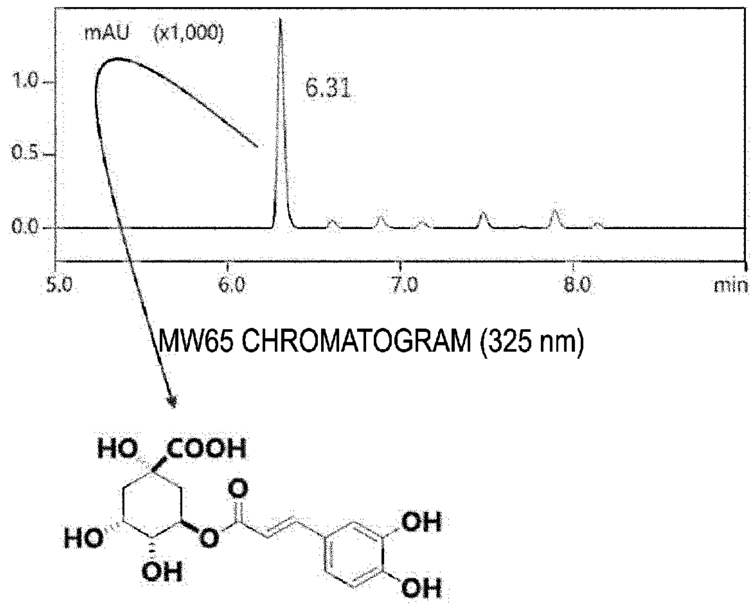


CHLOROGENIC ACID

Fig. 6

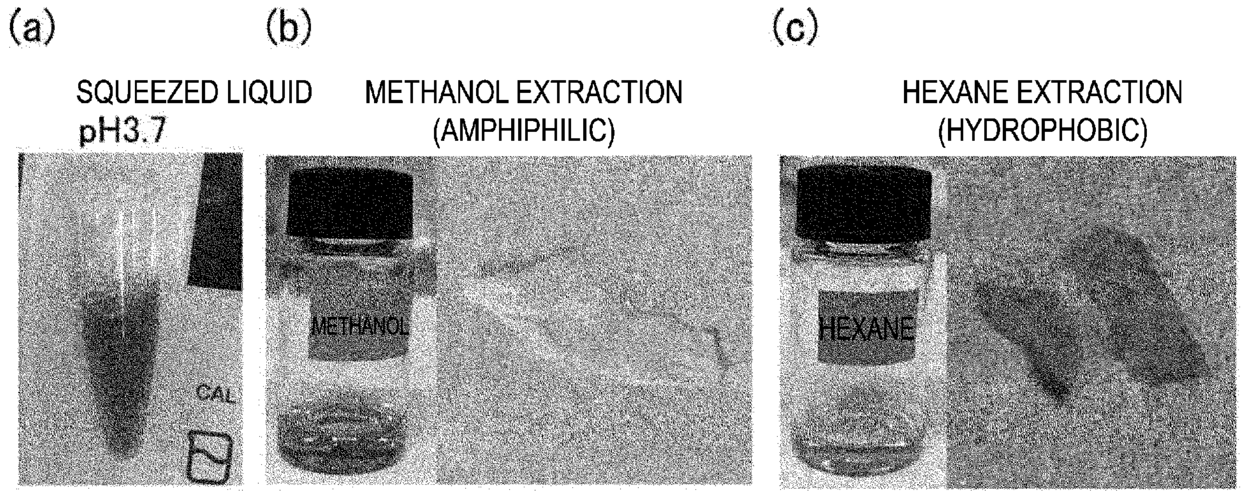
(a)
(b)
(c)
SQUEEZED LIQUID
pH3.7
METHANOL EXTRACTION
(AMPHIPHILIC)
HEXANE EXTRACTION
(HYDROPHOBIC)
CAL
METHANOL
HEXANE

Fig. 7

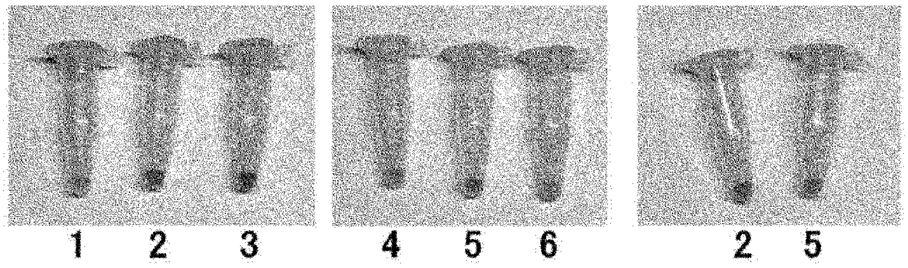


50 mM PHOSPHATE BUFFER

| | pH | ANTHOCYANIN | COPIGMENT | METAL ION |
|---|---|---|---|---|
| 1 | 4.4 to 4.9 | 0.5 mM CYANIDIN (AGLYCONE) | – | – |
| 2 | 7.0 | 0.5 mM CYANIDIN (AGLYCONE) | – | – |
| 3 | 8.7 to 9.3 | 0.5 mM CYANIDIN (AGLYCONE) | – | – |
| 4 | 4.4 to 4.9 | 0.5 mM CYANIDIN (AGLYCONE) | 0.5 mM CHLOROGENIC ACID (3-CQA) | 0.1 mM ALUMINUM ION |
| 5 | 7.0 | 0.5 mM CYANIDIN (AGLYCONE) | 0.5 mM CHLOROGENIC ACID (3-CQA) | 0.1 mM ALUMINUM ION |
| 6 | 8.7 to 9.3 | 0.5 mM CYANIDIN (AGLYCONE) | 0.5 mM CHLOROGENIC ACID (3-CQA) | 0.1 mM ALUMINUM ION |

# Fig. 8

```
173-h1          GTCCCAATTTCCCAAGTCCCAGCGGAGTACTTGAGGCTGGCAAGTACCAACCCCCACGCT 60
173-h2          GTCCCAATTTCCCAAGTCCCAGCGGAGTACTTGAGACTGGCAAGTATCAACCCCCACGCT 60
MW65            GTCCCAATTTCCCAAGTCCCAGCAGACTACTTGACACTGGCAAGTACCAACCCCCAGGCT 60
                *********************** ** *******  ********** ********* ***

173-h1          CCAATTCGATTGGAATTTGTTGTTGTTGTACCAGCCAGACGTCGGTCTATCCACCACTGG 120
173-h2          CCAATTCGATTGGAATTCGTTGTTGTTGTACCAGCCGGACGTCGGTCTATCCACCACTGG 120
MW65            TCGATTCGATTGGAATTCGTTGTTGTTGTACCAGCCAGACGTCGGTCAACTCACCACTGG 120
                 * ************** ****************** ********** *  *********

173-h1          CCTCGCCATTAGTATCTTGAACAATTAACGTTTCAACCTCACGTCTCTCTTGACTGTCGT 180
173-h2          CCTCGCCATTAGTATCTTGAACAATTAACGTTTCAACCTCCTGTCTCTCTTGACTGTCGT 180
MW65            CCTCGCCATTA----------------ACGTTTCAACCACCCGTCTCTCTTGACTCTCGT 164
                ***********                *********** *  ************* ****

173-h1          GTGAGGATCCTTGCCTGGTCTTTATTAGAGGAACAAATATGTTCCAGAATATATAGTTAT 240
173-h2          GTGAGGATCCTTTCCTGGTCTTTATTAGAGGAACAAATATGTTGCAGAATATATAATTAT 240
MW65            GTGAGTATCCCTGCCTGGTCTTTATTAGAGGAACAAATATGTTGCAGA------------ 212
                ***** **** * ****************************** ****

173-h1          TAGTTAGGGATTAAAGAGGAATTACTCATCTTAATTAAATTACATGAAAATTTATCGTAA 300
173-h2          TAGTTAGGGATTAAAGAGGAATTACTCATCTTAATTAAATTACATGAAAATTTATCGTAA 300
MW65            ------------------GAATTACGCATCTTAATTAAATTACATGAAAATTTATCGTAA 254
                                  ******* **********************************

173-h1          AGACCTTAGAACATGTGTCAACCACCGTCCATCTTAGTTTCCTTCTCTCGAGCAGGTTAG 360
173-h2          AGACCTTAGAACATGTGTCAACCACCGTCCATCTTAGTTTCCTTCTCTCGAGCAGGTTAG 360
MW65            AGACCGAAGAACATGTGTCAACCACCATCTATCTTAGTTTCCTTCTCTCGAGCAGGATAG 314
                *****  ******************* ** ************************** ***

173-h1          TTACTATGATCCATTTTCTTCCACGTTGTTGGCGTTTCCTATAAATAGTAGTGTGACACA 420
173-h2          TTACTATGATCCATTTTCTTCCATGTTGTTGGCGTTTCCTATAAATAGTAGTGTGACACA 420
MW65            TTACTATGATCTATTTTCTTCCATGTTGTTGGCGTTTCCTATAAATAGTAGTGTGACACA 374
                *********** *********** ************************************

173-h1          AATGAGATCTCATATTGATTTTCATCTTCTTCTGTAAAAAGAAAAAAAAAAATGGCCACC 480
173-h2          AATGAGATCTCATATTGATTTTCAACTTCTTCTGTAAAAAGAAAAAAAAAA-TGGCCACC 479
MW65            AATGAGATTTCATATTGATTTTCAACTTCTTCTGTAAAAAGAAAAAAAAAAATGGCCACC 434
                ******** *************** ************************** ********

173-h1          AAAAACTTATGTGAAAAGCATGTTGCTGTTCTTACATTTCCATTTGCATCCCATCCTGGC 540
173-h2          AAAAACTTATGTGAAAAGCATGTTGCTGTTCTTACATTTCCATTTGCATCCCATCCTGGC 539
MW65            AAAAACTTATGTGAAAAGCATGTTGCTGTTCTTACATTTCCATTTGCATCCCATCCTGGC 494
                ************************************************************

173-h1          CTTCTGTTAGGCCTTCTACATAGATTAGTTAAGGCTGCACCACATGTGACTTTCTCTTTT 600
173-h2          CTTCTGTTAGGCCTTCTACATAGATTAGTTAAGGCTGCACCACATGTGACTTTCTCTTTT 599
MW65            CTTCTGTTAGGCCTTCTACATAGATTAGTTAAGGCTGCACCACATGTGACTTTCTCTTTT 554
                ************************************************************

173-h1          C 601
173-h2          C 600
MW65            C 555
                *
```

# Fig. 9

```
173-h1    TAATCCATTACTGGTTGTCGGTTGACTTGATCTTTGATATACGGACGGTATTGGCTTTGC 60
173-h2    TAATCAATTACTGGTTGTCGGTTGACTTGATCTTTGATATACGGACGGTATTGGCTTTGC 60
MW65      TAATCAATTACTGGT-GTCGGTTGACTTGATCNTTGATATACGGACGGTATTGGCTTTGC 59
          ***** ********* **************** ***************************

173-h1    AGTTTGCACCAGAGAGGAAATAAAGAGA-------------------------------- 88
173-h2    AGTTTGCACCAGAGAGGAAATAAAAAGA-------------------------------- 88
MW65      AGTTT-CACCAGAGAGGAAATAAAGAGATTAGTGTCTCTTCCTTAGTTCCTGATTAGTTT 118
          ***** ****************** ***

173-h1    ------------------------------------------------------------
173-h2    ------------------------------------------------------------
MW65      GTGTATATTTTTCAATTTAGTTCCTGATTAGTTTGTGCATATTTTTCAATTTACATACTT 178

173-h1    ------------------------------------------------------------
173-h2    ------------------------------------------------------------
MW65      AACGTATTATGAACGATCTCTTAAAATTCAGAATTAATTGAATGAAACTTAAATGGCTGA 238

173-h1    ---------------------ACAGAAGGAAAGAACGGTCGTTTGTAAATGTTCAACCCC 127
173-h2    ---------------------ACAGAAGGAAAGAACGGTCGTTTGTAAATGTTCAACCCC 127
MW65      ATTATAAAAATAAAAAAAAAAACAGAAGGAAAGAACGGCCGTTTGTAAATGTTCAACCCC 298
                               ***************** *********************

173-h1    CATGTCAGGTGGCTCGTGCCAGCAATCGGGTGGTTAATGTGTATTT-GATCAGAAGCGTT 186
173-h2    CATGTCAGGTGGCTCGTGCCAGCAACCGGGTGGTTAATGTGTATTT-GATCAGAAGCGTT 186
MW65      CATGTCAGGTGGCTCGTGCCAGCAACCGGGTGGTTAATGTGTATTTTGATCAGAAGCGTT 358
          ************************* ******************** *************

173-h1    GTCTTGGCCAGGTTTGTACTAGTAAATTACACCACATAAATTATGGTGTATGTATTAAAA 246
173-h2    GTCTTGGCCAGGTTTGTACTAGTAAATTACACCACATAAATTATGGTGTATGTATTAAAA 246
MW65      ATCTTGGCCAGGTTTGTACTAGTATATTACACCACATAAATTATGGTGCATGTATTGAGA 418
           *********************** *********************** ******* * *

173-h1    TTGGGCGAAAAAACAAGGTCCGTGAACTTTCCGGATTCTAATGCATTAAGAAGATAATAT 306
173-h2    TTGGGCGAAAAAACAAGGTCCGTGAACTTTCCGGATTCTAATGCATTAAGAAGATAATAT 306
MW65      TTGGGCGAAAAAACAAGGTCCGTGAACTTTACCGATTCTAATGCATTACTAAGATAATAT 478
          ****************************** * ***************  **********

173-h1    ACTACTATTAGTTGTCTAAAATCAATTGGACATTTTTATTTTTCTTTTAAACATACTTGA 366
173-h2    ACTACTATTAGTTGTTTAAAATCAATTGGACATTTTTATTTTTCTTTTAAACATACTTGA 366
MW65      ACTACTATTACTTGTCTAAAATCAATTGGGCATTTTTATTCTTCTTTTAAACATACTTGA 538
          ********** **** ************* ********** *******************

173-h1    ATGGCGGACCACTTACCAGACCATAGTTCTAAGACTTGACCGGCAGACACCAACCAATTT 426
173-h2    ATGGCGGACCACTTACCAGACCATAGTTCTAAGACTTGACCGGCAGACACCAACCAATTT 426
MW65      ATGGCGTACCACTTGCCAGACCATAGTTGTAAGACTTGACCGGTAGGCACCAACCAATTT 598
          ****** ******* ************* ************** ** *************

173-h1    AGGAGTCCTCCATAAATAATCGGTGATGTGCCATATAGGGTCACCAAAAAATTGGTTTAA 486
173-h2    AGGAGTCCTCCATAAATAATCGGTGATGTGCCATATAGGGTCACCAAAAAATTGGTTTAA 486
MW65      AGGAGTCCTCCATAAATAATCGGTGATGTGCCATATTGGGTCACCAAAAAATTGGTTTAA 658
          ************************************ ***********************

173-h1    GCTTTGTCATCTCCCCAAATGGAGTACGAGTACG------ACTGCGAAAATTGCTTGAGC 540
173-h2    GCTTTGTCATCTCCCCAAATGGAGTACCAGTACG------ACTGCGAAAATTGCTTGAGC 540
MW65      GCTTTGTCATTTCCCCAAATGGAGTACGAGTACGCGTACGACTGCGAAAATTGCTTGAGC 718
          ********** **************** ******      ********************

173-h1    ATTGTGATGTACCCATGGTTTGGAATGGGACATCTCACGGCATTTCTTCATCTGTCCAAT 600
173-h2    ATTGTGATGTACCCATGGTTTGGAATGGGACATCTCACGGCATTTCTTCATCTGTCCAAT 600
MW65      ATTGTGATGTACCCATGGTTTGGAATGGGACATCTCACGGCATTTCTTCATCTGTCCAAT 778
          ************************************************************

173-h1    AAGCTTGCTCAGAAAGGCCATAGAATTTTCTTCATTTTGCCAACAAAATCGCAATCCAAG 660
173-h2    AAGCTTGCTCAGAAAGGCCATAGAATTTTCTTCATTTTGCCAACAAAATCGCAATCCAAG 660
MW65      AAGCTTGCTCAGATAGGCCATAAAATTTTCTTCATTTTGCCAACAAAATCGCAATCCAAG 838
          ************* ******** *************************************

173-h1    TTGGAAAACTTCAATCTTCATCCACACCGCATCAAGTTCA 700
173-h2    TTGGAAAACTTCAATCTTCATCCACACCGCATCAAGTTCA 700
MW65      TTGGAAAACTTCAATCTTCATCCACACCGCATCAAGTTCA 878
          ****************************************
```

Fig. 10

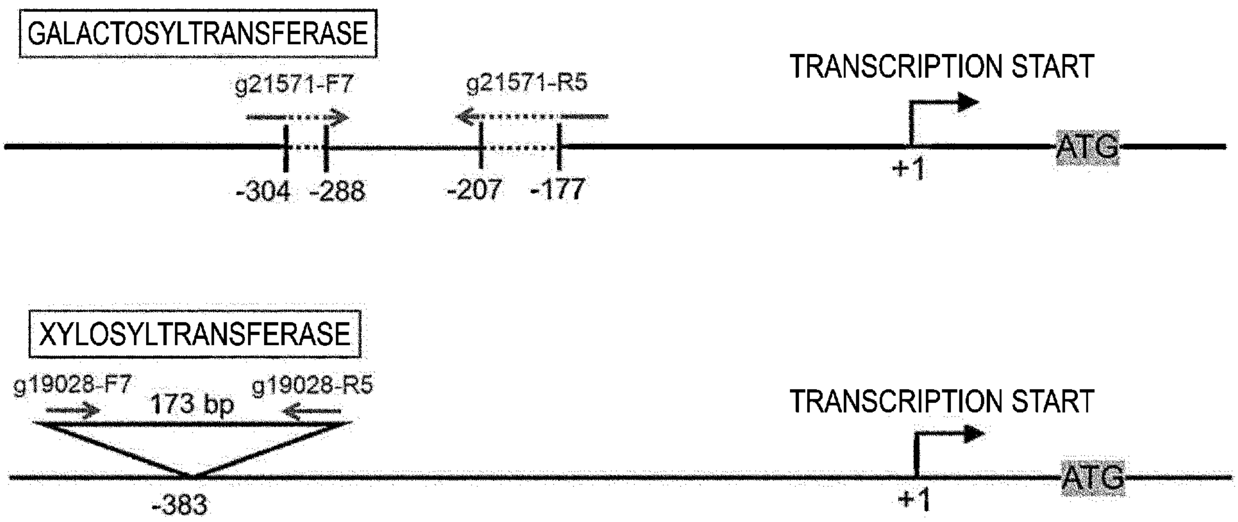
GALACTOSYLTRANSFERASE
g21571-F7
g21571-R5
TRANSCRIPTION START
-304
-288
-207
-177
+1
ATG
XYLOSYLTRANSFERASE
g19028-F7
173 bp
g19028-R5
TRANSCRIPTION START
-383
+1
ATG

Fig. 11

(a) GALACTOSYLTRANSFERASE MARKER

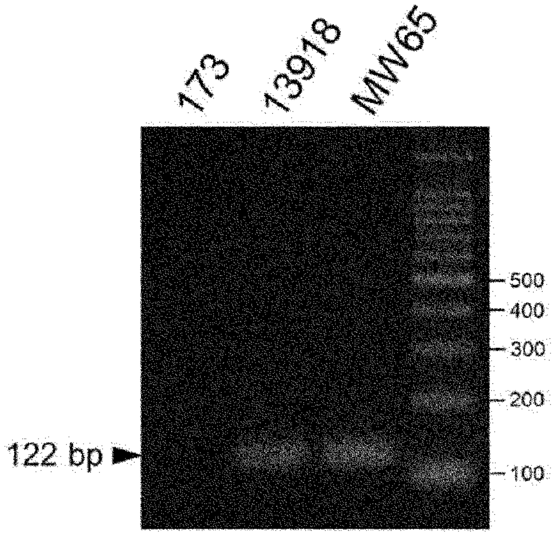


(b) XYLOSYLTRANSFERASE MARKER

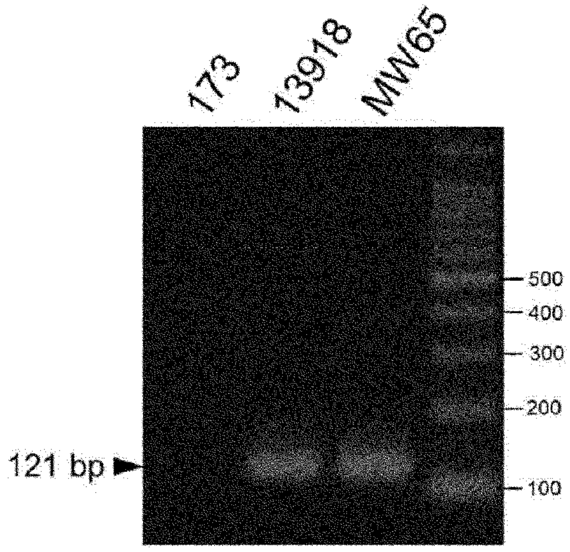

Fig. 12

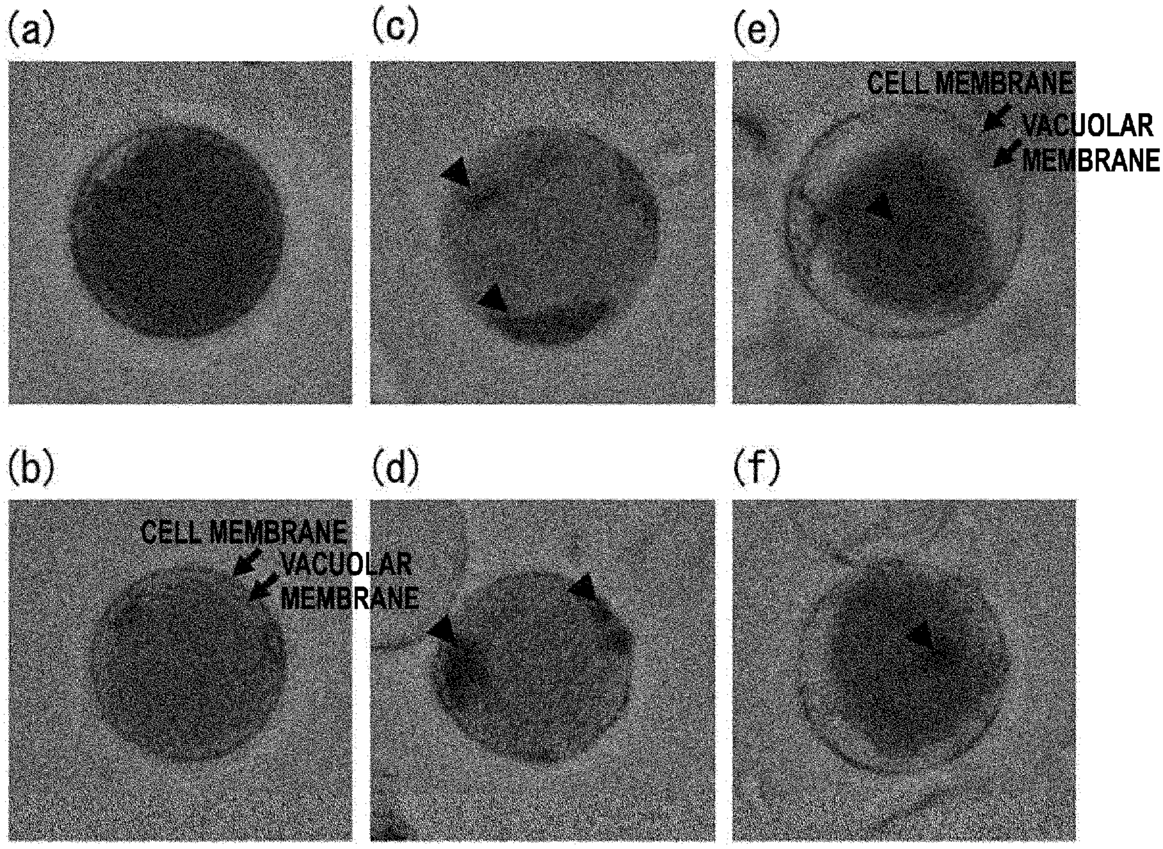


RED VARIETY 173

VACUOLAR LOCALIZATION

HYBRID 13918

VACUOLAR LOCALIZATION, BLUE GRANULAR STRUCTURE IN CYTOPLASM

BLUE VARIETY MW65

CYTOPLASMIC LOCALIZATION, GRANULAR STRUCTURE

**INTERNATIONAL SEARCH REPORT**

International application No.
**PCT/JP2024/034732**

**A. CLASSIFICATION OF SUBJECT MATTER**

***A01H 1/00***(2006.01)i; ***A01H 5/00***(2018.01)i; ***A01H 6/00***(2018.01)i; ***A01H 6/08***(2018.01)i; ***A01H 6/82***(2018.01)i; ***C12N 5/10***(2006.01)i; *C12N 15/54*(2006.01)n
FI: A01H1/00 A ZNA; A01H5/00 A; A01H5/00 Z; A01H6/00; A01H6/08; A01H6/82; C12N5/10; C12N15/54

According to International Patent Classification (IPC) or to both national classification and IPC

**B. FIELDS SEARCHED**

Minimum documentation searched (classification system followed by classification symbols)

A01H1/00-17/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII), CAplus/MEDLINE/EMBASE/BIOSIS (STN), UniProt/GeneSeq, GenBank/EMBL/DDBJ/GeneSeq

**C. DOCUMENTS CONSIDERED TO BE RELEVANT**

| Category* | Citation of document, with indication, where appropriate, of the relevant passages | Relevant to claim No. |
|---|---|---|
| X<br><br>A | WO 2017/065302 A1 (SUNTORY FLOWERS LIMITED) 20 April 2017 (2017-04-20)<br>claim 1, paragraph [0033] | 21-24<br><br>1-20 |
| A | WO 2017/169699 A1 (NAT AGRICULTURE & FOOD RES ORGANIZATION) 05 October 2017 (2017-10-05)<br>claim 1 | 1-24 |
| A | WO 2021/065749 A1 (SUNTORY HOLDINGS LTD.) 08 April 2021 (2021-04-08)<br>claims, examples | 1-24 |
| A | WO 2020/203940 A1 (SUNTORY HOLDINGS LTD.) 08 October 2020 (2020-10-08)<br>claims, examples | 1-24 |
| A | WO 2019/069946 A1 (SUNTORY HOLDINGS LTD.) 11 April 2019 (2019-04-11)<br>claims, examples | 1-24 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **29 November 2024** | **10 December 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (July 2022)

## INTERNATIONAL SEARCH REPORT

International application No.
**PCT/JP2024/034732**

**C. DOCUMENTS CONSIDERED TO BE RELEVANT**

| Category* | Citation of document, with indication, where appropriate, of the relevant passages | Relevant to claim No. |
|---|---|---|
| A | WO 2012/036290 A1 (SUNTORY HOLDINGS LTD.) 22 March 2012 (2012-03-22) claims, examples | 1-24 |
| A | WO 2008/156214 A1 (INTERNATIONAL FLOWER DEVELOPMENTS PROPRIETARY LIMITED) 24 December 2008 (2008-12-24) claims, examples | 1-24 |
| A | JP 2010-22365 A (TOYAMA PREFECTURE) 04 February 2010 (2010-02-04) claims, examples | 1-24 |
| A | JP 2012-511916 A (INTERNATIONAL FLOWER DEVELOPMENTS PROPRIETARY LIMITED) 31 May 2012 (2012-05-31) claims, examples | 1-24 |

Form PCT/ISA/210 (second sheet) (July 2022)

**INTERNATIONAL SEARCH REPORT**

International application No.
**PCT/JP2024/034732**

**Box No. I** **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed.
   - b. ☐ furnished subsequent to the international filing date for the purposes of international search (Rule 13*ter*.1(a)),
     - ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/JP2024/034732**

| Patent document cited in search report | Publication date (day/month/year) | Patent family member(s) | Publication date (day/month/year) |
|---|---|---|---|
| WO 2017/065302 A1 | 20 April 2017 | US 2018/0317418 A1<br>claim 1, paragraph [0092]<br>EP 3363283 A1<br>CN 107072164 A | |
| WO 2017/169699 A1 | 05 October 2017 | US 2020/0325486 A1<br>claim 1<br>CN 108777947 A | |
| WO 2021/065749 A1 | 08 April 2021 | US 2023/0220357 A1<br>claims, examples<br>EP 4039804 A1<br>CN 114450404 A | |
| WO 2020/203940 A1 | 08 October 2020 | US 2022/0186240 A1<br>claims, examples | |
| WO 2019/069946 A1 | 11 April 2019 | US 2020/0283782 A1<br>claims, examples<br>CN 111386342 A | |
| WO 2012/036290 A1 | 22 March 2012 | US 2013/0347143 A1<br>claims, examples<br>EP 2617283 A1<br>KR 10-2013-0114093 A<br>CN 103249301 A | |
| WO 2008/156214 A1 | 24 December 2008 | US 2010/0287668 A1<br>claims, examples<br>EP 2159282 A1<br>CN 101688198 A<br>KR 10-2010-0029104 A | |
| JP 2010-22365 A | 04 February 2010 | (Family: none) | |
| JP 2012-511916 A | 31 May 2012 | US 2011/0321184 A1<br>claims, examples<br>WO 2010/069004 A1<br>EP 2358870 A1 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017169699 A
- JP 2021175402 A


### Non-patent literature cited in the description


- **TATSUZAWA, F.** *Biochem. Systemat. Ecol.*, 2021, vol. 99, 104347
- **MACZ-POP, G. A**. *Food Chem.*, 2006, vol. 94, 448-456
- **OYAMA, K.** *J. Agric. Food Chem.*, 2015, vol. 63, 7630-7635
- Computational Molecular Biology. Oxford University Press, 1988
- Biocomputing: Informatics and Genome Projects. Academic Press, 1993
- Computer Analysis of Sequence Data: Part I. Human Press, 1994
- **VON HEINJE, G**. Sequence Analysis in Molecular Biology. Academic Press, 1987
- Sequence Analysis Primer. M-Stockton Press, 1991